(19) **Europäisches Patentamt**
**European Patent Office**
**Office européen des brevets**

(11) **EP 1 797 814 B1**

(12) **EUROPÄISCHE PATENTSCHRIFT**

(45) Veröffentlichungstag und Bekanntmachung des Hinweises auf die Patenterteilung:
**13.03.2013 Patentblatt 2013/11**

(51) Int Cl.:
***A61B 3/10*** (2006.01)

(21) Anmeldenummer: **06023207.1**

(22) Anmeldetag: **08.11.2006**

(54) **Verfahren und Vorrichtung zur Bestimmung des Abstandes zu einem Messpunkt auf einer Gewebefläche des Auges**

Method and device for determining the distance to a measuring point on a tissue surface of the eye

Procédé et dispositif pour déterminer la distance à un point de mesure sur une surface de tissu d'un oeil

(84) Benannte Vertragsstaaten:
**CH ES IT LI**

(30) Priorität: **13.12.2005 DE 102005059923**

(43) Veröffentlichungstag der Anmeldung:
**20.06.2007 Patentblatt 2007/25**

(73) Patentinhaber: **Oculus Optikgeräte GmbH**
**35582 Wetzlar (DE)**

(72) Erfinder: **Köst, Gert**
**30171 Hannover (DE)**

(74) Vertreter: **von den Steinen, Axel**
**advotec.**
**Patent- und Rechtsanwälte**
**Georg-Schlosser-Strasse 6**
**35390 Giessen (DE)**

(56) Entgegenhaltungen:
**WO-A-2005/058152     DE-A1- 3 324 341**
**DE-A1- 3 429 062     US-A- 4 261 367**
**US-A- 5 347 328     US-B1- 6 231 186**

**Beschreibung**

Die Erfindung betrifft ein Verfahren und eine Vorrichtung zur Bestimmung des Abstandes zu einem Messpunkt auf einer Gewebefläche des Auges.

Derartige Abstandsmessungen sind in der Augenheilkunde von großer Bedeutung. Insbesondere die Achslänge des Auges, das heißt der Abstand zwischen Hornhaut und Netzhaut ist bei vielen heilkundlichen Verfahren von großer Wichtigkeit. Die Messung des Abstandes zu Messpunkten auf Gewebeflächen im Inneren des Auges bietet allerdings erhebliche Schwierigkeiten, da mit den eingesetzten Messwerkzeugen das Auge selbst nicht verletzt werden darf.

Die bekannten Messverfahren zur Vermessung des Auges sind deshalb außerordentlich aufwendig und ungenau.

Aufgabe der vorliegenden Erfindung ist es deshalb, ein neues Verfahren zur Bestimmung von Abständen am und/oder im Auge vorzuschlagen, das mit relativ einfachen Mitteln und schnell durchgeführt werden kann und dabei genaue Messergebnisse liefert. Weiter ist es Aufgabe der vorliegenden Erfindung eine Vorrichtung zur Durchführung des erfindungsgemäßen Verfahrens vorzuschlagen.

Diese Aufgaben werden durch ein Verfahren und eine Vorrichtung nach der Lehre der unabhängigen Hauptansprüche gelöst.

Vorteilhafte Ausführungsformen der Erfindung sind Gegenstand der Unteransprüche.

Das erfindungsgemäße Verfahren beruht auf dem Grundgedanken, zur Messung des Abstandes zu einem Messpunkt auf einer Gewebefläche des Auges Lichtimpulse einzusetzen, die an der entsprechenden Gewebefläche reflektiert werden. Da sich der Lichtimpuls und der an der Gewebefläche reflektierte Lichtimpuls (Reflektorlichtimpuls) mit Lichtgeschwindigkeit, das heißt sehr schnell ausbreitet, ist allerdings die unmittelbare Messung der Laufzeit des Lichts vom Zeitpunkt der Aussendung bis zum Empfang des Reflektorlichtimpulses an einem Lichtsensor kaum möglich. Insbesondere sind solche unmittelbaren Messungen der Laufzeit mit hohen Messfehlern behaftet, so dass die Messergebnisse in der Praxis nicht weiter verwendet werden können.

Erfindungsgemäß wird deshalb vorgeschlagen, einen Schwingkreis aufzubauen. In diesem Schwingkreis wird der jeweils nächste Lichtimpuls der Impulslichtquelle (Folgelichtimpuls) dadurch ausgelöst, dass am Lichtsensor ein Reflektorlichtimpuls des vorhergehenden Folgelichtimpulses detektiert wird. Das heißt mit anderen Worten, jeder Folgelichtimpuls wird an der Gewebefläche des Auges reflektiert und der so entstandene Reflektorlichtimpuls am Lichtsensor detektiert. Durch die Detektion des Reflektorlichtimpulses wird der jeweils nächste Folgelichtimpuls der Impulslichtquelle ausgelöst. Um den Schwingkreis zu Beginn in Schwingung zu versetzen, wird von der Impulslichtquelle ein Anfangslichtimpuls erzeugt.

Mit geeigneten Messmitteln wird die Frequenz des Schwingkreises, das heißt, die Anzahl von Folgelichtimpulsen pro Zeiteinheit direkt oder indirekt gemessen. Die Frequenzmessung ist dabei mit relativ einfachen Messmitteln möglich.

Da die spezifische Lichtgeschwindigkeit, mit der sich das Impulslicht im Medium entlang des Strahlengangs zwischen Impulslichtquelle und dem Messpunkt beziehungsweise dem Messpunkt und dem Lichtsensor bekannt ist, kann aus der direkt oder indirekt gemessenen Schwingfrequenz des Schwingkreises die Länge des Strahlengangs, ausgehend von der Impulslichtquelle, über den Messpunkt auf der Gewebefläche des Auges zum Lichtsensor aus der gemessen Schwingfrequenz abgeleitet werden. Denn in der gemessenen Schwingfrequenz ist die Information enthalten, welche Zeit notwendig ist, um die entsprechende Anzahl von Folgelichtimpulsen, die dann jeweils die volle Länge des Strahlengangs durchlaufen haben, zu ergeben. Wird diese Laufzeit auf einen einzigen Folgelichtimpuls normiert und diese auf einen Folgelichtimpuls normierte Laufdauer mit der spezifischen Lichtgeschwindigkeit multipliziert, ergibt sich daraus die Länge des Strahlengangs.

Dabei ist es besonders vorzugswürdig, wenn Impulslichtquelle und Lichtsensor in der zur Durchführung des Verfahrens vorgesehenen Vorrichtung derart angeordnet sind, dass die Länge X des Strahlengangs zwischen der Impulslichtquelle und dem Messpunkt genau der Länge Y des Strahlengangs zwischen dem Messpunkt und dem Lichtsensor entspricht. Dies bedeutet mit anderen Worten, dass die Laufzeit beziehungsweise Lauflänge des Folgelichtimpulses ausgehend von der Impulslichtquelle bis zur Gewebefläche exakt der Lauflänge und Laufzeit des Reflektorlichtimpulses, ausgehend von der Gewebefläche des Auges zum Lichtsensor entspricht. Auf diese Weise kann die durch Auswertung der gemessenen Frequenz ermittelte Länge des Strahlengangs einfach halbiert werden, um auf diese Weise zu dem gesuchten Abstand zwischen Impulslichtquelle und dem Messpunkt auf der Gewebefläche des Auges zu gelangen. Die Länge des Strahlenganges zwischen der Impulslichtquelle und dem Messpunkt auf der Gewebefläche des Auges lässt sich dabei nach der Formel berechnen $X = 0{,}5 \times c \times (1/F1)$. Soweit die Länge des Strahlenganges des Folgelichtimpulses nicht exakt der Länge des Strahlenganges des Reflektorlichtimpulses entspricht, muss der Faktor 0,5 durch weitere Korrekturfaktoren, die addiert beziehungsweise multipliziert werden, korrigiert werden. Der Faktor $1/F1$ ergibt sich daraus, dass die Laufzeit des Folgelichtimpulses, die der Folgelichtimpuls ausgehend von der Impulslichtquelle bis zur Gewebefläche des Auges benötigt, durch den Kehrwert der gemessenen Schwingfrequenz ersetzt wird.

Mit dem erfindungsgemäßen Messprinzip, bei der aus der Messung der Schwingfrequenz, mit der der Schwingkreis aus Impulslichtquelle, Lichtsensor und Steuerelektronik schwingt, können die jeweiligen Abstände zu den jeweiligen Gewebeflächen einfach aus den gemessenen Schwingfrequenzen abgeleitet werden. Dabei ist jedoch zu beachten, dass die abgeleiteten Ergebnisse für die Länge des Strahlengangs einen systematischen Messfehler enthalten, der durch die Verzögerung des Signallaufes in der Steuerelektronik verursacht wird. Nach Auftreffen des Reflektorlichtimpulses auf dem Lichtsensor benötigt der Lichtsensor für die Detektion des Lichtsignals, die Steuerelektronik für die Übertragung des detektierten Signals und die Impulslichtquelle für die Auslösung des nächsten Folgelichtimpulses nämlich eine bestimmte Verzögerungszeit, die bauteilbedingt ist. Diese Verzögerungszeit der verwendeten Bauteile ist dabei zudem größeren Schwankungen unterworfen, da die Bauteile sich beispielsweise bei unterschiedlichen Temperaturen unterschiedlich verhalten. Um eine aufwendige Kalibrierung, mit der die durch die Verzögerungszeit verursachten Messfehler eliminiert oder zumindest abgeschätzt werden können, zu vermeiden, ist vorgesehen, dass die Frequenzmessung nacheinander bei Reflektion der Lichtimpulse an zwei verschiedenen Messpunkten durchgeführt wird, wobei die Messpunkte auf Gewebeflächen des Auges liegen.

Aus den beiden Messwerten F1 und F2 kann dann die Längendifferenz XD, durch die sich die Längen X1 und X2 der beiden Strahlengänge bei Reflektion der Lichtimpulse am ersten Messpunkt und bei Reflektion der Lichtimpulse am zweiten Messpunkt unterscheiden, abgeleitet werden. Die aus den gemessenen Schwingungsfrequenzen F1 und F2 abgeleitete Längendifferenz XD wird dabei nicht mehr durch die bauteilbedingten Verzögerungszeiten verfälscht, da dieser Messfehler in beiden gemessenen Frequenzen gleich groß enthalten ist und sich bei der Differenzbildung entsprechend heraushebt.

Soweit Impulslichtquelle und Lichtsensor wiederum derart angeordnet sind, dass die Lauflängen der Folgelichtimpulse exakt der Länge der Reflektorlichtimpulse entspricht, kann die Längendifferenz XD nach der Formel $XD = 0{,}5 \times c \times (1/F2 - 1/F1)$ berechnet werden.

Gemäß der Erfindung liegt der zweite Messpunkt ebenfalls auf einer zweiten Gewebefläche des Auges. In diesem Fall repräsentiert die abgeleitete Längendifferenz XD gerade den Abstand zwischen den beiden Gewebeflächen im Auge. Der erste Messpunkt kann also beispielsweise auf der Netzhaut des Auges platziert werden und der zweite Messpunkt auf der Vorderseite der Hornhaut. Auf diese Weise könnte dann aus der Längendifferenz XD in einfacher Weise die Achsenlänge des Auges zwischen Hornhaut und Netzhaut ermittelt werden.

Soweit in dem zur Durchführung des Verfahrens vorgesehenen Gerät zusätzlich eine zweite unabhängige Messeinrichtung, insbesondere ein Triangulationsmessgerät, vorhanden ist, mit der der Abstand zwischen einem Bezugspunkt im Gerät und einem Messpunkt auf einer Gewebefläche an der Außenseite des Auges, insbesondere an der Vorderseite der Hornhaut, bestimmt werden kann, kann durch entsprechende Differenzbildung der Abstände zum jeweiligen Bezugspunkt, der Abstand zwischen den einzelnen Messpunkten zum Messpunkt an der Außenseite des Auges ermittelt werden.

Um die Frequenzmessung bei Reflektion der Lichtimpulse an den verschiedenen Gewebeflächen des Auges durchführen zu können, müssen die Reflektorlichtimpulse, die an den verschiedenen Gewebeflächen des Auges reflektiert worden sind, voneinander differenziert werden. Eine Möglichkeit zur Differenzierung der Reflektorlichtimpulse an den einzelnen Gewebeflächen liegt darin, dass die Lichtintensität der verschiedenen Reflektorlichtimpulse berücksichtigt wird. Dies beruht darauf, dass ein Folgelichtimpuls an den verschiedenen Gewebeflächen des Auges, beispielsweise an der Vorderseite oder der Rückseite der Hornhaut beziehungsweise an der Vorderseite oder Rückseite der natürlichen Linse beziehungsweise an der Netzhaut unterschiedlich stark reflektiert werden. Die stärksten Reflektionslichtimpulse erhält man dabei bei Reflektion an der Netzhaut. In dem Messsignalspektrum der gemessenen Lichtintensitäten können dann verschiedene Triggerschwellen definiert werden. Je nachdem ob die einzelnen Triggerschwellen überschritten beziehungsweise unterschritten sind, kann dann aus dem empfangenen Signal der Reflektorlichtimpuls des an einer bestimmten Gewebefläche reflektierten Lichts selektiert werden.

Bei Selektion der verschiedenen Reflektorlichtimpulse der verschiedenen Gewebeflächen am oder im Auge, ist es besonders vorteilhaft, wenn die Lichtintensität der Lichtimpulse zwischen den verschiedenen Messgängen verändert wird. Dadurch ist es möglich, da mit einer einzigen Triggerschwelle verschiedene Reflektorlichtimpulse selektiert werden, da durch die Abschwächung beziehungsweise Verstärkung der Lichtintensität auch die verschiedenen Reflektorlichtimpulse jeweils entsprechend abgeschwächt oder verstärkt werden.

Die Veränderung der Lichtintensität kann selbstverständlich dadurch erfolgen, dass die Impulslichtquelle entsprechend mit geänderten Parametern angesteuert wird. Da sich durch jede Veränderung in der Impulslichtquelle beziehungsweise dem Lichtsensor Eigenschaftsänderungen ergeben können, die die bauteilbedingten Verzögerungszeiten verändern, ist es jedoch vorzuziehen, dass die Lichtintensität durch Einbringung eines Abdunklungselements, insbesondere durch Verstellung eines Graukeilelements, in den Strahlengang der Lichtimpulse verändert wird. Auf diese Weise bleiben die bauteilbedingten Verzögerungszeiten zwischen den einzelnen Messgängen immer konstant und die Änderung der Lichtintensität wird durch eine entsprechende Abschattung mit dem Abdunklungselement rein passiv bewirkt.

In welcher Art die Frequenzmessung durchgeführt wird, ist grundsätzlich beliebig. Insbesondere kann die Frequenzmessung durch Einsatz einer Zeitmesseinrichtung und einem Impulszählgerät geleistet werden.

Mit der Zeitmesseinrichtung wird die Messdauer eines Messganges bestimmt, wohingegen das Impulszählgerät die während der Messdauer abgegebenen Lichtimpulse zählt. Aus der Relation zwischen den gezählten Lichtimpulsen und der gemessenen Messdauer ergibt sich dann die jeweilige Frequenz.

Alternativ dazu ist es auch denkbar, dass die Messdauer jedes Messganges als Festwert vorgegeben ist und die während der Messdauer abgegebenen Lichtimpulse gezählt werden. Eine Zeitmesseinrichtung zur variablen Zeitmessung kann auf diese Weise entfallen.

Alternativ dazu ist es auch möglich, dass die abzugebenen Lichtimpulse als Festwert vorgegeben sind und die zur Abgabe dieser festen Anzahl von Lichtimpulsen erforderliche Messdauer mit der Zeitmesseinrichtung gemessen wird. Ein Impulszählglied zur variablen Zählung der abgegebenen Lichtimpulse kann auf diese Weise entfallen.

Alternativ beziehungsweise additiv zur Änderung der Lichtintensität der abgegebene Lichtimpulse zur Reflektion der an den verschiedenen Gewebeflächen reflektierten Reflektorlichtimpulse ist es auch denkbar, dass ein konfokaler Optikfilter im Strahlengang der Reflektorlichtimpulse angeordnet wird. Derartige konfokale Optikfilter haben die Eigenschaft, dass nur Lichtsignale den Optikfilter passieren können, die im Nahbereich einer bestimmten Messebene reflektiert werden. Alle Lichtsignale, die nicht im Nahbereich der vorgegebenen Messebene des Optikfilters reflektiert werden, werden durch den Optikfilter abgefiltert. Der konfokale Optikfilter kann dabei beispielsweise aus mehreren Linsen und einer Lochblende bestehen. Durch entsprechende Fokussierung der Lichtsignale in den Linsen wird erreicht, dass die nur die an der vorgegebenen Messebene reflektierten Lichtsignale von den Linsen derart fokussiert werden, dass die Lochblende passiert werden kann. Alle nicht fokussierten Lichtsignale, die damit außerhalb der Messebene liegen, werden von der Lochblende ausgefiltert.

Durch den konfokalen Optikfilter ist es somit möglich, dass am Lichtsensor jeweils nur solche Reflektorlichtsignale empfangen werden, die in einer bestimmten Messebene reflektiert wurden. Die Messebene des konfokalen Optikfilters kann dabei durch Einstellung des Optikfilters verschoben werden. Bei der Vermessung des Auges kann dann der Optikfilter kontinuierlich oder schrittweise derart verstellt werden, dass die Messebene ausgehend von einer Anfangsstellung bis zu einer Endstellung verstellt wird. Die Messebene bewegt sich dabei in Richtung einer Achse durch das Auge und schneidet das Auge in den verschiedenen Messpunkten auf den Gewebeflächen. Sobald am Lichtsensor ein Reflektorlichtsignal detektiert wird, wird durch entsprechende Frequenzmessung die Abstandsmessung durchgeführt. Durch entsprechende Verstellung der Messebene können somit nacheinander alle Gewebeflächen des Auges, an denen das Impulslicht reflektiert wird, mit ihrem jeweiligen Abstand bestimmt werden. Dazu ist es besonders vorteilhaft, wenn die Anfangsstellung oder Endstellung der Messebene vor beziehungsweise auf der Vorderseite der Hornhaut liegt. Wenn zugleich Anfangsstellung beziehungsweise Endstellung der Messebene auf oder hinter der Netzhaut liegt, kann im Ergebnis das gesamte Auge mit den verschiedenen Abständen zwischen den verschiedenen Gewebeflächen vermessen werden.

Weiterhin ist es besonders vorteilhaft, wenn die Stellung des Auges während der Frequenzmessung durch eine Fixierlichtquelle fixiert wird, um Messfehler durch unerwünschte Bewegungen des Auges zu verhindern.

Mit dem erfindungsgemäßen Verfahren können nicht nur Abstände zwischen verschiedenen Gewebeflächen des Auges bestimmt werden, sondern auch Höhenprofile in den einzelnen Gewebeflächen. Dazu wird nacheinander der Abstand zu mehreren nebeneinander auf der Gewebefläche des Auges liegende Messpunkte gemessen und aus den verschiedenen Messwerten das Höhenprofil abgeleitet.

Die Vermessung der verschiedenen, nebeneinander auf der Gewebefläche des Auges liegenden Messpunkte kann beispielsweise durch einen Scanbetrieb gewährleistet werden. Bei diesem Scanbetrieb wird der Messstrahl entsprechend einem vorgegebenen Raster nacheinander über die Gewebefläche des Auges geführt und die jeweiligen Abstände in den nebeneinander liegenden Messpunkten bestimmt. Aus den einzelnen Messpunkten ergibt sich dann das gewünschte Höhenprofil.

Alternativ zur Verwendung eines Scanbetriebes kann ein Höhenprofil auch dadurch erstellt werden, dass ein flächiger Impulslichtstrahl auf die Gewebefläche gerichtet wird. Die in den einzelnen Messpunkten reflektierten Impulslichtstrahlen werden dann von einem flächigen Lichtsensor, insbesondere einem CCD-Chip-Sensor, getrennt voneinander detektiert, so dass sich auf diese Weise das gewünschte Höhenprofil ergibt. Somit kann die Anzahl von Messgängen zur Erstellung des Höhenprofils erheblich verringert werden.

Welche Art von Höhenprofilen mit dem erfindungsgemäßen Verfahren vermessen werden, ist grundsätzlich beliebig. Nach einer bevorzugten Ausführungsform wird ein flächiges Höhenprofil des Nervenfaserkopfes am Ende des Sehnervs mit dem erfindungsgemäßen Verfahren abgeleitet. Dieses Höhenprofil des Nervenfaserkopfes kann Auskunft über bestimmte Krankheitsformen des Auges, insbesondere des Glaucoms, geben.

Weiter ist es besonders von Vorteil, wenn das Höhenprofil der Zäpfchen auf der Netzhaut mit dem erfindungsgemäßen Verfahren abgeleitet wird. Auf diese Weise können Schädigungen der Netzhaut, wie Sie beispielsweise durch Laserlichtbestrahlung verursacht werden, festgestellt werden.

Welche Gewebeflächen des Auges mit dem erfindungsgemäßen Verfahren in ihrem Abstand bestimmt werden, ist ebenfalls grundsätzlich beliebig. Besonders geeignet sind Messpunkte auf der Vorderseite beziehungsweise auf der Rückseite der Hornhaut, Messpunkten auf der Vorderseite beziehungsweise Rückseite der natürlichen Linse und/oder Messpunkte auf der Netzhaut.

Da sich die Lichtgeschwindigkeit in den unterschiedlichen Gewebearten des Auges unterschiedlich schnell ausbreitet, sollten diese aus spezifischen Ausbreitungsgeschwindigkeiten bei der Ableitung der gesuchten Abstände aus den gemessenen Frequenzen nach Möglichkeit berücksichtigt werden.

Alternativ dazu kann aber auch ein gemittelter Geschwindigkeitswert bei der Berechnung der Ableitung Verwendung finden.

Die Impulslichtquelle sollte bevorzugt in der Art einer Laserlichtquelle beziehungsweise in der Art einer Laserdiode, ausgebildet sein.

Als Lichtsensoren sind Fotodioden oder CCD-Chip-Sensors geeignet.

Verschiedene Ausführungsformen sind in den Zeichnungen schematisch dargestellt und werden nachfolgend beispielhaft erläutert.

Es zeigen:

**Fig. 1**: den prinzipiellen Aufbau einer Vorrichtung zur Vermessung des Abstandes zu einem Messpunkt auf einer Gewebefläche des Auges;

**Fig. 2**: die Vorrichtung gemäß **Fig. 1** kombiniert mit einer unabhängigen Messeinrichtung zur Vermessung des Abstandes zu einem Messpunkt auf einer zweiten Gewebefläche an der Außenseite des Auges;

**Fig. 3**: die Vorrichtung gemäß **Fig. 1** mit einem zweiten Strahlengang zur Vermessung des Abstandes zu einem zweiten Messpunkt auf einer zweiten Gewebefläche des Auges;

**Fig. 4**: eine Vorrichtung zur Vermessung von Abständen am Auge;

**Fig. 5**: die Vorrichtung gemäß **Fig. 4** bei Vermessung des Abstandes zu einem Messpunkt auf einer Referenzfläche;

**Fig. 6**: Vorrichtung gemäß **Fig. 4** bei Vermessung des Abstandes zu einer zweiten Gewebefläche des Auges;

**Fig. 7**: die Vorrichtung gemäß **Fig. 1** bei ergänzendem Einsatz einer Abdunklungseinrichtung;

**Fig. 8**: die Vorrichtung gemäß **Fig. 7** nach Verstellung der Abdeckungseinrichtung;

**Fig. 9**: die Intensitäten der Reflektorlichtimpulse an drei verschiedenen Gewebeflächen bei Einstellung der Abdunklungseinrichtung gemäß **Fig. 7**;

**Fig. 10**: die Intensitäten der Reflektorlichtimpulse bei Einstellung der Abdunklungseinrichtung gemäß **Fig. 8**;

**Fig. 11**: eine Vorrichtung zur Vermessung von Abständen am Auge;

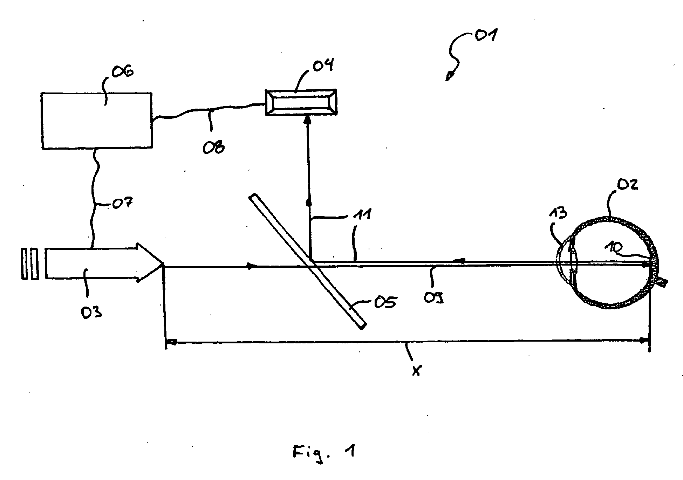

**Fig. 1** stellt den grundsätzlichen Aufbau einer Vorrichtung 01 zur Vermessung des Abstandes X zum Auge 02 dar. Die Vorrichtung 01 umfasst eine Impulslichtquelle 03, beispielsweise eine Laserdiode, einen Lichtsensor 04, beispielsweise eine Fotodiode, einen Strahlenteiler 05 und eine Steuerelektronik 06 mit integrierter Recheneinheit. Die Steuerelektronik 06 ist über Leitungen 07 und 08 mit der Impulslichtquelle 03 und dem Lichtsensor 04 verbunden.

Zu Beginn der Messung erzeugt die Steuerelektronik 06 einen Anfangsimpuls, so dass die Impulslichtquelle 03 einen Anfangslichtimpuls 09a abgibt. Dieser Anfangslichtimpuls 09a wird an den verschiedenen Gewebeflächen des Auges 02, insbesondere an der Netzhaut 10, reflektiert und der Reflektionslichtimpuls 11 nach Umlenkung am Strahlenteiler 05 auf den Lichtsensor 04 gelenkt. Impulslichtquelle 03, Strahlenteiler 05 und Lichtsensor 04 sind dabei derart angeordnet, dass die Länge des Strahlenganges von der Impulslichtquelle 03 zur Netzhaut 10 genauso lang wie der Strahlengang von der Netzhaut 10 zum Lichtsensor 04 ist.

Sobald am Lichtsensor 04 ein Reflektionslichtimpuls 11 detektiert wird, wird das über die Leitung 08 an die Steuerelektronik 06 weitergemeldet. Nach Eingang diese Detektionssignals löst die Steuerelektronik 06 über die Leitung 07 einen weiteren Lichtimpuls, nämlich einen Folgelichtimpuls 09b, der Impulslichtquelle 03 aus. Auch der Folgelichtimpuls 09b wird wieder an der Netzhaut 10 reflektiert und als Reflektionslichtimpuls 11 am Lichtsensor 04 detektiert. Die Detektion des Reflektionslichtimpuls 11 löst wiederum einen weiteren Folgelichtimpuls 09b aus.

Da sich der Abstand des Auges 02 zur Vorrichtung 01 im Wesentlichen nicht verändert, schwingt der aus Impulslichtquelle 03, der Netzhaut 10, dem Lichtsensor 04 und der Steuerelektronik 06, gebildete Schwingkreis anschließend mit einer bestimmten Frequenz F1. Diese Frequenz F1 entspricht dabei dem Kehrwert der Laufzeit, die das Licht, ausgehend von der Impulslichtquelle 03 bis zur Netzhaut 10 und zurück von der Netzhaut 10 bis zum Lichtsensor 04 benötigt. Außerdem geht in die gemessene Frequenz F1 noch die Verzögerungszeit ein, die bauteilbedingt benötigt wird, um ab dem Eingang des Reflektionslichtimpulses 11 beim Lichtsensor 04 den darauffolgenden Folgelichtimpuls 09b an der Impulslichtquelle abzugeben. Soweit diese Verzögerungszeit ausreichend klein ist, beziehungsweise bei entsprechender Kalibrierung der Vorrichtung 01 kann die bauteilbedingte Verzögerungszeit vernachlässigt beziehungsweise korrigiert werden.

Da die Länge des Strahlengangs von der Impulslichtquelle 03 bis zur Netzhaut und zurück von der Netzhaut bis zum Lichtsensor 04 gleich groß ist, gilt die Formel X=0,5 x c (spezifische Lichtgeschwindigkeit) x t (Laufzeitdauer für eine Schwingung). Die Laufzeitdauer t für eine Schwingung kann dann durch den Kehrwert der gemessenen Frequenz 1/F ersetzt werden, so dass sich der Abstand X nach der Formel berechnet X=0,5 x c x 1/F1.

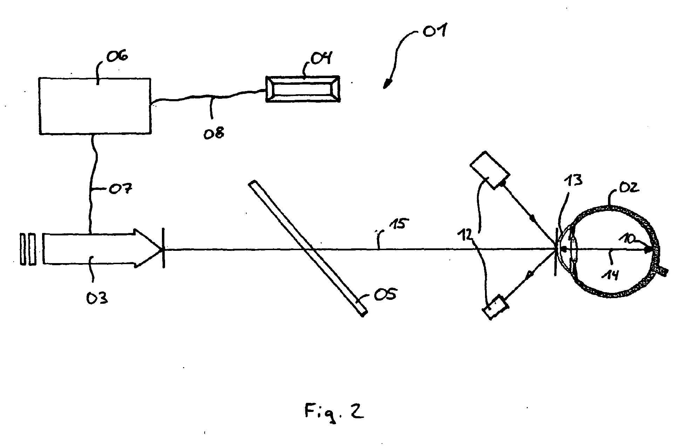

**Fig. 2** zeigt, wie der durch Frequenzmessung ermittelte Abstand X im Rahmen einer Augenuntersuchung weiter genutzt werden kann. Wird nach der in **Fig. 2** dargestellten Ausführungsform in die Vorrichtung 01 eine zweite, unabhängige Messeinrichtung 12, nämlich ein Triangulationsmessgerät, integriert, mit dem der Abstand der Vorderseite der Hornhaut 13 bestimmt werden kann, so liegen im Endergebnis Messergebnisse für den Abstand der Vorrichtung 01 zur Netzhaut 10 und zum Abstand der Vorrichtung 01 zur Hornhaut 13 vor, so dass durch entsprechende Subtraktion die Achslänge 14 des Auges bestimmt werden kann.

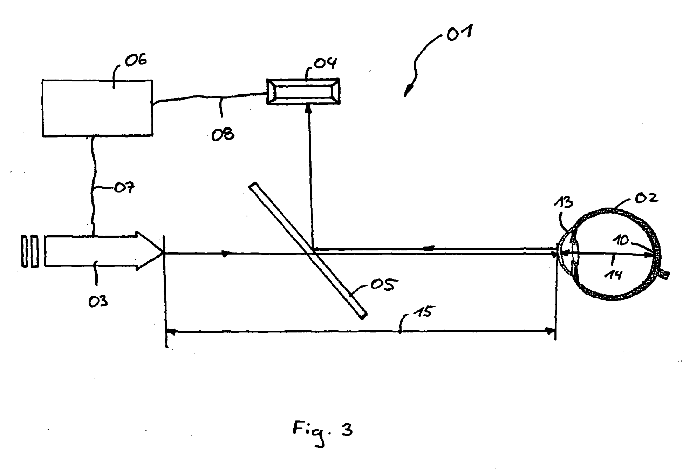

**Fig. 3** verdeutlicht eine erfindungsgemäße Methode zur Bestimmung der Achslänge 14. Bei dieser Methode wird zunächst, entsprechend dem in **Fig. 1** dargestellten Vorgehen der Abstand X zwischen Impulslichtquelle 03 und Netzhaut 10 bestimmt. Anschließend wird wie in **Fig. 3** dargestellt ein Schwingkreis aus der Impulslichtquelle 03, der Hornhaut 13, dem Lichtsensor 04 und der Steuerelektronik 06 aufgebaut und die sich dabei ergebende Schwingfrequenz F2 gemessen. Ohne die Berechnung des Abstandes 15 zwischen der Impulslichtquelle 03 und der Hornhaut 13 wird die Achslänge 14 als Längendifferenz XD gemäß der Formel abgeleitet XD=0,5 x c x (1/F2 - 1/F1). Die durch die Verzögerungszeit der Bauteile verursachten Messfehler werden durch diese Mess- und Berechnungsmethode ohne weiteres eliminiert, da die entsprechenden Messfehler jeweils gleich groß in der Messung der Frequenz F1 als auch in der Frequenz F2 enthalten sind und sich damit eliminieren.

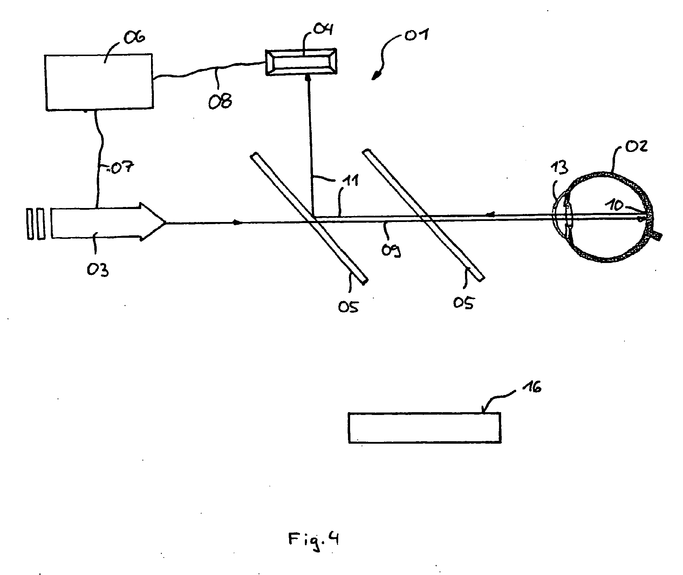

**Fig. 4** bis **Fig. 6** stellen eine nicht erfindungsgemäße Variante zur Eliminierung der durch die Verzögerungszeit der Bauteile bedingten Messfehler dar. Bei dieser Variante wird, wie in **Fig. 4** zunächst dargestellt, wiederum ein Schwingkreis mit einer ersten Gewebefläche, im dargestellten Beispiel mit der Netzhaut 13 aufgebaut und die sich dabei ergebende Schwingfrequenz F1 gemessen.

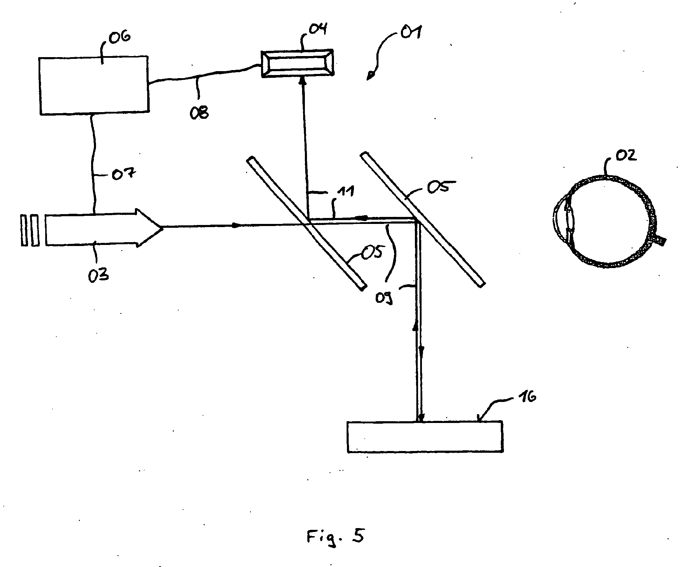

Anschließend wird, wie in **Fig. 5** dargestellt ein Schwingkreis bei der die Reflektion des Lichtes an einer Referenzfläche 16 aufgebaut und die sich dabei ergebende Schwingfrequenz F2 gemessen wird. Aus den beiden gemessenen Frequenzen F1 und F2 kann dann wiederum die Längendifferenz XD, die die unterschiedliche Länge der beiden in **Fig. 4** und **Fig. 5** dargestellten Strahlengänge repräsentiert, gemäß der Formel XD=0,5 x c x (1/F2 - 1/F1) abgeleitet werden. Dadurch werden wiederum die bauteilbedingten Verzögerungszeiten eliminiert, da sie in beiden Frequenzen gleich groß repräsentiert sind.

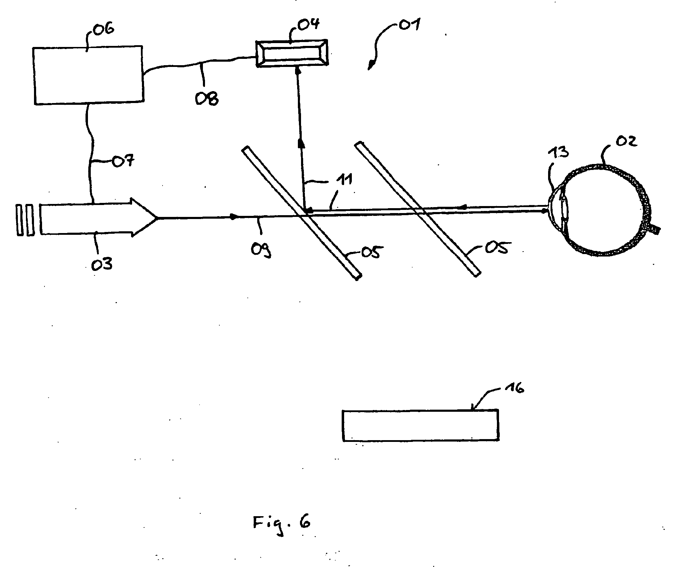

Anschließend wird, wie in **Fig. 6** dargestellt, ein Schwingkreis mit einer zweiten Gewebefläche, im dargestellten Beispiel mit der Hornhaut 13, aufgebaut und die sich dabei ergebende Schwingfrequenz F3 gemessen. Die Längendifferenz XD2 zwischen dem in **Fig. 6** dargestellten Strahlengang und dem in **Fig. 5** dargestellten Strahlengang bei Reflektion an der Referenzfläche 16 ergibt sich dann wiederum aus der Formel XD2 = 0,5 x c x (1/F2 - 1/F3). Wird nun XD1 von XD3 subtrahiert, so ergibt sich die Achsenlänge 14 unmittelbar.

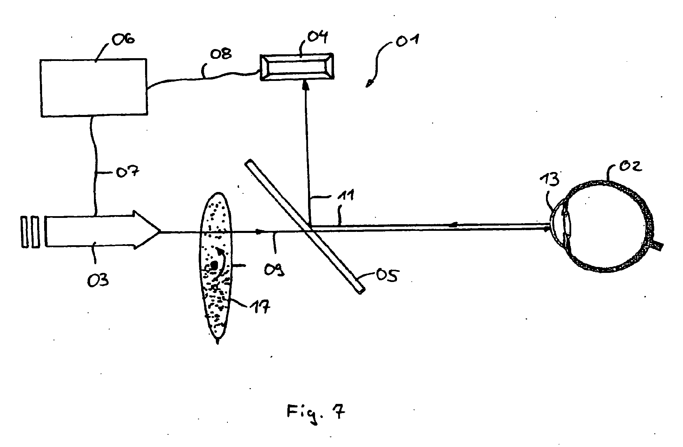

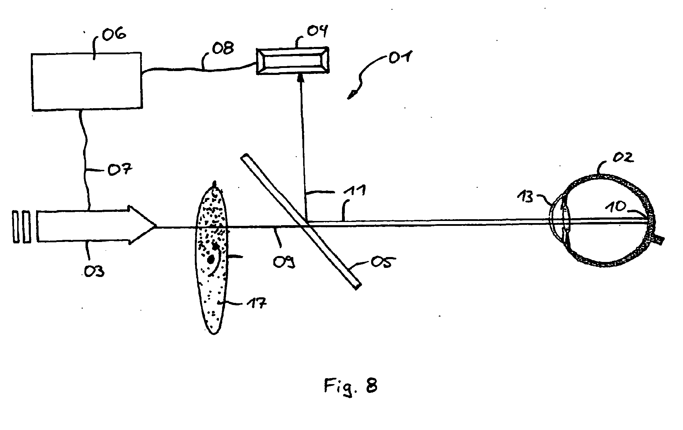

**Fig. 7** bis **Fig. 8** stellen schematisch eine Variante dar, mit denen die Schwingkreise zwischen den verschiedenen Gewebeflächen des Auges umgeschaltet werden können. Um diese zu erreichen, wird in den Strahlengang eine Abdunklungseinrichtung 17, nämlich ein drehbar gelagerter Graukeil, eingeschaltet. Durch Drehen des Graukeils 17 kann das von der Impulslichtquelle 03 ausgesendete Impulslicht 09 unterschiedlich stark abgeschwächt werden. In **Fig. 7** und **Fig. 8** ist der Strahlengang bei unterschiedlichen Stellwinkeln der Abdunklungseinrichtung 17 schematisch dargestellt, wobei die Abdunklung bei der in **Fig. 8** dargestellten Situation stärker als bei der in **Fig. 7** dargestellten Situation ist.

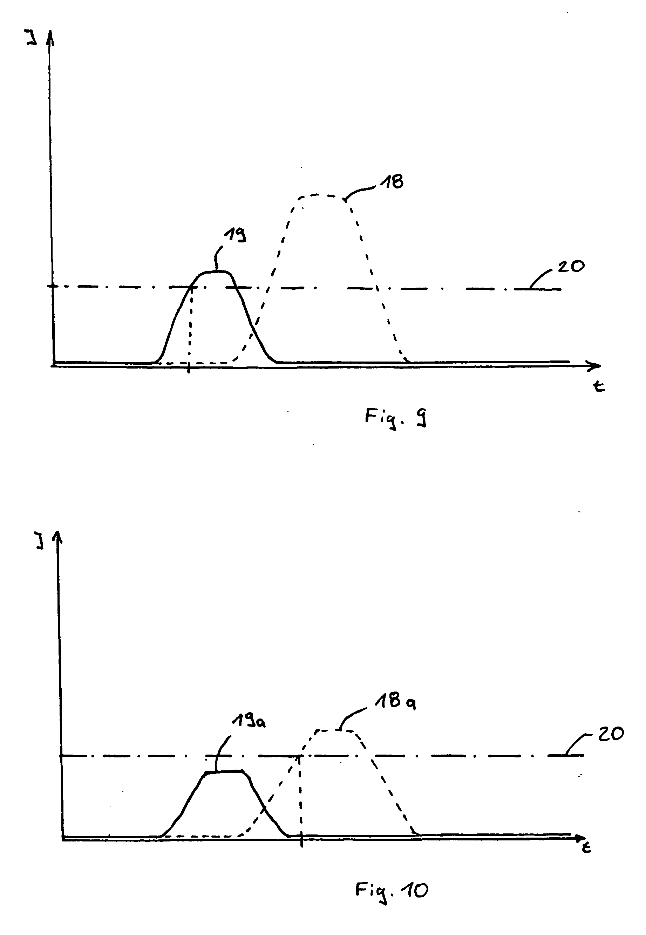

**Fig. 9** zeigt schematisch die am Lichtsensor 04 empfangenen Intensitäten der Reflektorlichtimpulse, die an den verschiedenen Gewebeflächen des Auges reflektiert sind. Den stärksten Reflektionslichtimpuls erzeugt die Reflektion an der Netzhaut 10, der in der **Fig. 9** als Reflektionslichtkurve 18 strichliniert dargestellt ist. Die Reflektionslichtkurve 19 des an der Hornhaut reflektierten Lichts ist in ihrer Intensität schwächer und liegt zeitlich aufgrund des geringeren Abstandes der Hornhaut zur Impulslichtquelle 03 etwas früher als das Maximum der Reflektionslichtkurve 18. Zur Differenzierung des Reflektionslichtes zwischen den beiden Gewebeflächen, nämlich zwischen der Netzhaut und der Hornhaut, wird in der Steuerungseinrichtung 06 eine Triggerschwelle 20 definiert. Dies bedeutet mit anderen Worten, dass der nächste Folgelichtimpuls der Impulslichtquelle 03 von der Steuerungseinrichtung 06 immer dann ausgelöst wird, wenn die Triggerschwelle 20 vom Sensorsignal überschritten wird. Nach Auslösung eines Folgelichtimpulses ist die Auslösung eines nächsten Folgelichtimpulses für eine gewisse Zeitspanne bauartbedingt oder durch entsprechende Maßnahmen ausgeschlossen. Bei dem in **Fig. 9** dargestellten Signalverlauf bedeutet dies, dass aufgrund der relativ niedrigen Triggerschwelle 20 das Folgelichtsignal jeweils vom Reflektionslicht 19 der Hornhaut ausgelöst wird, so dass sich der in **Fig. 7** dargestellte Schwingkreis bei Reflektion des Lichtimpulses an der Hornhaut 13 aufbaut. Das Reflektionslicht 18 der Netzhaut dagegen, wird aufgrund der bauartbedingten Totzeit nach Auslösung des jeweils nächsten Folgelichtimpulses nicht berücksichtigt.

Die in **Fig. 10** dargestellten Intensitäten der Reflektionslichter 18a und 19a entsprechen der Situation nach Abdunklung des Strahlenganges durch Verdrehen des Graukeils 17. Aufgrund der geringeren Lichtintensitäten des auf die Netzhaut 10 beziehungsweise auf die Hornhaut 13 auftreffenden Lichtimpulses, sind auch die Reflektionslichtkurven 18 und 19 entsprechend herabgesetzt. Das Reflektionslicht 19 des an der Hornhaut reflektierten Lichts reicht dabei nicht mehr aus, um die Triggerschwelle 20 zu überschreiten, so dass sich nun der in **Fig. 8** dargestellte Schwingkreis bei Reflektion des Lichtes an der Netzhaut 10 aufbaut.

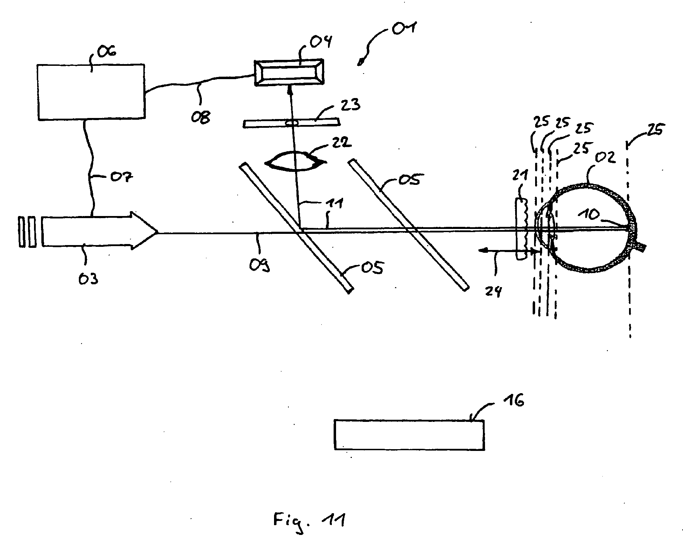

**Fig. 11** zeigt eine Alternative zur Selektion der verschiedenen Gewebeflächen im Auge, die bei der Frequenzmessung jeweils Berücksichtigung finden. Bei dieser Variante wird im Strahlengang des Reflektionslichtes ein konfokaler Optikfilter angeordnet, der aus einem Mikrolinsenarray 21, einer Linse 22 und einer Lochblende 23 besteht. Durch Verschieben des Mikrolinsenarrays 21 entsprechend des angedeuteten Stellwegs 24 können jeweils verschiedene Messebenen 25 im Auge fokussiert werden. Durch die zusätzliche Anordnung 22 und der Lochblende 23 im Strahlengang des Reflektionslichtes wird dafür gesorgt, dass jeweils nur die durch das Mikrolinsenarray 21 fokussierten Messebenen auf den Lichtsensor 04 abgebildet werden, wohingegen alle anderen Bereiche des Auges 02 ausgeblendet sind. Bei der Vermessung des Auges 02 kann nun das Mikrolinsenarray 21, ausgehend von einer Anfangsstellung langsam von vorne an das Auge angenähert oder vom Auge weggefahren werden, so dass dadurch die Messebene 25 kontinuierlich durch das Auge 02 verschoben wird. Immer wenn eine der Gewebeflächen im Auge, an denen das Impulslicht reflektiert wird, mit den Mikrolinsen des Arrays 21 fokussiert ist, das heißt, wenn die Netzebene 25 durch die entsprechende Gewebefläche verläuft, wird der in dieser Gewebefläche reflektierte Reflektionslichtimpuls auf den Lichtsensor 04 übertragen. Alle anderen Reflektionslichtimpulse, die an den anderen Gewebeflächen reflektiert wurden, werden durch die Lochblende 23 ausgefiltert. Sobald am Lichtsensor 24 ein Reflektionslichtimpuls detektiert wird, baut sich ein Schwingkreis auf und die entsprechende Schwingfrequenz wird gemessen. Sobald die Schwingfrequenz festgestellt ist, wird das Mikrolinsenarray 21 weiter verstellt und die Impulslichtquelle 03 gibt in regelmäßigen Zeitabständen kurz hintereinander Folgelichtimpulse ab, bis erneut ein Reflektionslichtimpuls empfangen wird und sich wiederum ein weiterer Schwingkreis aufbaut.

Aus den dabei nacheinander ermittelten Schwingfrequenzen F1 bis Fn können die verschiedenen Abstände zwischen den einzelnen Gewebeflächen 02 abgeleitet werden.

## Patentansprüche

1. Verfahren zur Bestimmung des Abstands zu einem Messpunkt auf einer Gewebefläche (10, 13) des Auges (02), wobei

a) mit einer Impulslichtquelle (03) ein Anfangslichtimpuls (09a) erzeugt und auf den Messpunkt des Auges gerichtet wird,
b) ein auf der Gewebefläche (10, 13) im Messpunkt reflektierte Reflektorlichtimpuls (11) mit einem Lichtsensor (04) aufgefangen wird,
**dadurch gekennzeichnet,**
**dass**
c) durch die Detektion des Reflektorlichtimpulses (11) am Lichtsensor (04) ein Folgelichtimpuls (09b) der Impulslichtquelle (03) ausgelöst wird,
d) die Impulslichtquelle (03) abhängig von der Detektion der Reflektorlichtimpulse (11), die vom jeweils vorhergehenden Folgelichtimpuls (09b) verursacht sind, weiter Folgelichtimpulse (09b) erzeugt und dabei mit einer Frequenz F1 schwingt,
e) die Frequenz F1 direkt oder indirekt gemessen wird, mit der die Impulslichtquelle (03) bei Reflektion der Lichtimpulse (09) an einem ersten Messpunkt auf einer ersten Gewebefläche (10, 13) des Auges schwingt,
und eine Frequenz F2 gemessen wird, mit der die Impulslichtquelle (03) bei Reflektion der Lichtimpulse (09) an einem zweiten Messpunkt auf einer zweiten Gewebefläche (10, 13) des Auges schwingt,
f) die Längendifferenz XD, durch die sich die Längen X1 und X2 der beiden Strahlengänge bei Reflektion der Lichtimpulse (09) am ersten Messpunkt und bei Reflektion der Lichtimpulse (09) am zweiten Messpunkt unterscheiden, mit den Längen X1 und X2 der Strahlengänge ausgehend von der Impulslichtquelle (03) über die Messpunkte zum Lichtsensor (04) aus den Messwerten von F1, F2, und der spezifischen Lichtgeschwindigkeit c, mit der sich das Licht im Medium entlang des Strahlengangs ausbreitet, abgeleitet wird, wobei die Längendifferenz XD den Abstand zwischen den beiden Gewebeflächen (10, 13) repräsentiert, wobei die Längendifferenz XD zwischen den beiden Strahlengängen gemäß der Formel

$$XD = 0{,}5 \times c \times (1/F2 - 1/F1)$$

abgeleitet wird.

2. Verfahren nach Anspruch 1,
**dadurch gekennzeichnet,**
**dass** mit einem zweiten, unabhängigen Messverfahren der Abstand zwischen einem Bezugspunkt und einem Messpunkt auf einer Gewebefläche an der Außenseite des Auges, insbesondere an der an der Vorderseite der Hornhaut (13), bestimmt wird.

**3.** Verfahren nach Anspruch 2,
**dadurch gekennzeichnet,**
**dass** der mit dem zweiten, unabhängigen Messverfahren bestimmte Abstand zur Voreinstellung und/oder Kontrolle des Frequenzmessverfahren eingesetzt wird.

**4.** Verfahren nach Anspruch 2,
**dadurch gekennzeichnet,**
**dass** mit dem Frequenzmessverfahren der Abstand zwischen dem Bezugspunkt und einem reflektierendem Messpunkt auf einer Gewebefläche im Inneren des Auges (02), insbesondere auf der Netzhaut (10), bestimmt wird, wobei durch Subtraktion des mit dem zweiten, unabhängigen Messverfahren bestimmten Abstandes von dem mit dem Frequenzmessverfahren bestimmten Abstand der Abstand abgeleitet wird der zwischen einer Gewebefläche (10) im Inneren des Auges (02) und einer Gewebefläche (13) an der Außenseite des Auges (02) vorhanden ist.

**5.** Verfahren nach einem der Ansprüche 1 bis 4,
**dadurch gekennzeichnet,**
**dass** der Reflektorlichtimpuls (11) am Lichtsensor (04) dadurch detektiert wird, dass die vom Lichtsensor (04) gemessene Lichtintensität zumindest eine vorgegebene Triggerschwelle (20) übersteigt und/oder unterschreitet.

**6.** Verfahren nach Anspruch 5,
**dadurch gekennzeichnet,**
**dass** das Frequenzmessverfahren in zumindest zwei Messgängen wiederholt wird, wobei die Lichtintensität der Lichtimpulse (09) zwischen den verschiedenen Messgängen verändert wird.

**7.** Verfahren nach Anspruch 6,
**dadurch gekennzeichnet,**
**dass** die Lichtintensität durch Einbringung eines Abdunkelungselements (17), insbesondere durch Verstellung eines Graukeilelements, in den Strahlengang der Lichtimpulse (09) verändert wird.

**8.** Verfahren nach einem der Ansprüche 1 bis 7,
**dadurch gekennzeichnet,**
**dass** bei der Frequenzmessung zur Bestimmung der Frequenz F die Messdauer mit einer Zeitmesseinrichtung gemessen und die während der Messdauer abgegebenen Lichtimpulse (09) mit einer Impulszählglied gezählt werden.

**9.** Verfahren nach einem der Ansprüche 1 bis 7,
**dadurch gekennzeichnet,**
**dass** bei der Frequenzmessung zur Bestimmung der Frequenz F die Messdauer als Festwert vorgeben ist und die während der festen Messdauer abgegebenen Lichtimpulse (09) mit dem Impulszählglied gezählt werden.

**10.** Verfahren nach einem der Ansprüche 1 bis 7,
**dadurch gekennzeichnet,**
**dass** bei der Frequenzmessung zur Bestimmung der Frequenz F die abzugebenden Lichtimpulse (09) als Festwert vorgegeben ist und die zur Abgabe der als Festwert vorgegeben Lichtimpulse (09) erforderliche Messdauer mit der Zeitmesseinrichtung gemessen wird.

**11.** Verfahren nach einem der Ansprüche 1 bis 10,
**dadurch gekennzeichnet,**
**dass** im Strahlengang der Reflektorlichtimpulse (11) ein konfokaler Optikfilter (21, 22, 23) angeordnet wird, wobei der Optikfilter (21, 22, 23) Reflektorlichtimpulse (11), die im Nahbereich einer bestimmten Messebene (25) des Optikfilters reflektiert werden, passieren lässt und wobei der Optikfilter (21, 22, 23) Reflektorlichtimpulse 11), die außerhalb des Nahbereichs der Messebene (25) reflektiert werden, abfiltert.

**12.** Verfahren nach Anspruch 11,
**dadurch gekennzeichnet,**
**dass** der Optikfilter (21, 22, 23) kontinuierlich oder schrittweise derart verstellt wird, dass die Messebene (25) ausgehend von einer Anfangsstellung entlang der Längsachse des Strahlengangs bis zu einer Endstellung verstellt wird, wobei bei Detektion von Reflektorlichtimpulsen (11) eine Frequenzmessung zur Bestimmung des Abstandes der reflektierenden Gewebefläche durchgeführt wird.

**13.** Verfahren nach Anspruch 12,
**dadurch gekennzeichnet,**
**dass** die Anfangsstellung oder Endstellung der Messebene vor oder auf der Vorderseite der Hornhaut (13) liegt.

**14.** Verfahren nach Anspruch 12 oder 13,
**dadurch gekennzeichnet,**
**dass** die Anfangsstellung oder Endstellung der Messebene auf oder hinter der Netzhaut (10) liegt.

**15.** Verfahren nach einem der Ansprüche 1 bis 14,
**dadurch gekennzeichnet,**
**dass** die Stellung des Auges durch Betrieb einer Fixierlichtquelle fixiert wird.

**16.** Verfahren nach einem der Ansprüche 1 bis 15,
**dadurch gekennzeichnet,**
**dass** durch Vermessung des Abstandes mehrerer nebeneinander auf einer Gewebefläche (10, 13) des Auges (02) liegender Messpunkte ein flächiges Höhenprofil der Gewebefläche abgeleitet wird.

**17.** Verfahren nach Anspruch 16,
**dadurch gekennzeichnet,**
**dass** die Vermessung des Abstandes mehrerer nebeneinander auf einer Gewebefläche des Auges liegender Messpunkte durch Scannen der Gewebefläche erfolgt, wobei beim Scannen ein gebündelter Impulslichtstrahl nacheinander auf nebeneinander liegende Messpunkte gerichtet wird.

18. Verfahren nach Anspruch 16,
**dadurch gekennzeichnet,**
**dass** zur Vermessung des Abstandes mehrere nebeneinander auf einer Gewebefläche des Auges liegender Messpunkte ein flächiger Impulslichtstrahl auf die Gewebefläche gerichtet wird, wobei die in einzelnen Messpunkten reflektierten Impulslichtstrahlen von einem flächigen Lichtsensor, insbesondere einem flächigen CCD-Chip-Sensor, getrennt voneinander detektiert werden.

19. Verfahren nach einem der Ansprüche 16 bis 18,
**dadurch gekennzeichnet,**
**dass** ein flächiges Höhenprofil des Nervenfaserkopfes am Ende des Sehnervs abgeleitet wird.

20. Verfahren nach einem der Ansprüche 16 bis 18,
**dadurch gekennzeichnet,**
**dass** ein flächiges Höhenprofil der Zäpfchen auf der Netzhaut abgeleitet wird.

21. Verfahren nach einem der Ansprüche 1 bis 20,
**dadurch gekennzeichnet,**
**dass** zumindest ein reflektierender Messpunkt auf einer Gewebefläche an der Außenseite des Auges (02), insbesondere an der Vorderseite der Hornhaut (13), liegt.

22. Verfahren nach einem der Ansprüche 1 bis 20,
**dadurch gekennzeichnet,**
**dass** zumindest ein reflektierender Messpunkt auf einer Gewebefläche im Inneren des Auges (02) liegt.

23. Verfahren nach Anspruch 22,
**dadurch gekennzeichnet,**
**dass** ein Messpunkt auf der Netzhaut (10) liegt.

24. Verfahren nach Anspruch 22,
**dadurch gekennzeichnet,**
**dass** ein Messpunkt auf der Rückseite der Hornhaut (13) und/oder auf der Vorderseite des Linsenkörpers und/oder auf der Rückseite des Linsenkörpers liegt.

25. Verfahren nach einem der Ansprüche 1 bis 24,
**dadurch gekennzeichnet,**
**dass** der Abstand zwischen einem Messpunkt auf der Vorderseite der Hornhaut (13) und einem Messpunkt auf der Netzhaut (10) bestimmt und als Achslänge (14) des Auges (02) ausgegeben wird.

26. Verfahren nach einem der Ansprüche 1 bis 25,
**dadurch gekennzeichnet,**

**dass** als spezifischen Lichtgeschwindigkeit c ein gemittelter Geschwindigkeitswert eingesetzt wird, der aus der Ausbreitungsgeschwindigkeit des Lichts in den verschieden Medien entlang des Strahlengangs zwischen der Impulslichtquelle (03) und dem Messpunkt gemittelt ist.

27. Verfahren nach einem der Ansprüche 1 bis 26,
**dadurch gekennzeichnet,**
**dass** die spezifischen Lichtgeschwindigkeit c aus der Ausbreitungsgeschwindigkeit des Lichts in Luft und der gemittelten Ausbreitungsgeschwindigkeit des Lichts im Auge (02) gemittelt ist.

28. Verfahren nach einem der Ansprüche 1 bis 27,
**dadurch gekennzeichnet,**
**dass** gemittelten Ausbreitungsgeschwindigkeit des Lichts im Auge (02) aus der Ausbreitungsgeschwindigkeit in den in den verschieden Medien des Auges (02), insbesondere in der Hornhaut (13) und/oder in der Vorderkammer und/oder im Linsengewebe und/oder im Kammerwasser gemittelt ist.

29. Vorrichtung zur Bestimmung des Abstands zu einem Messpunkt auf einer Gewebeflächen (10, 13) des Auges (02), umfassend

- zumindest eine Impulslichtquelle (03) zur Erzeugung von Lichtimpulsen (09), die auf den Messpunkt gerichtet werden können,
- und zumindest einem Lichtsensor (04), zur Detektion von Reflektorlichtimpulsen (11), die im Messpunkt reflektiert wurden, **gekennzeichnet durch,**
- eine Steuerelektronik (06), die nach Detektion eines Reflektorlichtimpulses (11) weitere Folgelichtimpulse (09b) auslöst und dabei mit einer Frequenz F1 schwingt,
- eine Frequenzmesseinrichtung zur direkten oder indirekten Messung der Frequenz F1, die an einem ersten Messpunkt auf einer ersten Gewebefläche (10, 13) des Auges schwingt, und einer Frequenz F2 die an einem zweiten Messpunkt auf einer zweiten Gewebefläche (10, 13) des Auges schwingt,
- eine Recheneinheit, mit der die Längendifferenz XD, **durch** die sich die Längen X1 und X2 der beiden Strahlengänge bei Reflektion der Lichtimpulse (09) am ersten Messpunkt und bei Reflektion der Lichtimpulse (09) am zweiten Messpunkt unterscheiden, mit den Längen X1 und X2 der Strahlengänge ausgehend von der Impulslichtquelle (03) über die Messpunkte zum Lichtsensor (04) aus den Messwerten von F1, F2, und der spezifischen Lichtgeschwindigkeit c, mit der sich das Licht im Medium entlang des Strahlengangs ausbreitet, abgeleitet wird, wobei die Längendifferenz XD den Abstand zwischen den beiden Gewebeflächen (10, 13) repräsentiert, wobei die Längendifferenz XD zwischen den beiden Strahlengängen gemäß der Formel

$$XD = 0{,}5 \times c \times (1/F2 - 1/F1)$$

abgeleitet wird.

**30.** Vorrichtung nach Anspruch 29,
**dadurch gekennzeichnet,**
**dass** Impulslichtquelle (03) und Lichtsensor (04) derart in der Vorrichtung (01) angeordnet sind, dass die Länge X des Strahlengangs zwischen der Impulslichtquelle (03) und dem Messpunkt genau der Länge Y des Strahlengangs zwischen Messpunkt und Lichtsensor (04) entspricht.

**31.** Vorrichtung nach Anspruch 29 oder 30,
**dadurch gekennzeichnet,**
**dass** die Impulslichtquelle (03) in der Art einer Laserlichtquelle, insbesondere in der Art einer Laserdiode, ausgebildet ist.

**32.** Vorrichtung nach einem der Ansprüche 29 bis 31,
**dadurch gekennzeichnet,**
**dass** der Lichtsensor (04) in der Art einer Photodiode oder in der Art eines, insbesondere flächigen CCD-Chip-Sensors, ausgebildet ist.

**33.** Vorrichtung nach einem der Ansprüche 29 bis 32,
**dadurch gekennzeichnet,**
**dass** in die Vorrichtung (01) eine zweite, unabhängige Messeinrichtung (12) integriert ist, mit der der Abstand zwischen einem Bezugspunkt und einem Messpunkt auf einer Gewebefläche an der Außenseite des Auges, insbesondere an der Vorderseite der Hornhaut, bestimmt werden kann.

**34.** Vorrichtung nach Anspruch 33,
**dadurch gekenntzeichnet,**
dass die zweite, unabhängige Messeinrichtung (12) in der Art eines Trianguluationsmessgeräts ausgebildet ist.

**35.** Vorrichtung nach einem der Ansprüche 29 bis 34,
**dadurch gekennzeichnet,**
**dass** das Ausgangssignal des Lichtsensors (04) in einem Lichtintensitätstriggerelement ausgewertet wird, wobei der Reflektorlichtimpulse (11) am Lichtsensor (04) dadurch detektiert wird, dass die vom Lichtsensor gemessene Lichtintensität eine vorgegebene Triggerschwelle (20) übersteigt oder unterschreitet.

**36.** Vorrichtung nach einem der Ansprüche 29 bis 35,

**dadurch gekennzeichnet,**
**dass** die Vorrichtung (01) ein Abdunkelungselement (17), insbesondere einen Graukeilelement, umfasst, mit dem die Lichtintensität der Lichtimpulse (09) verändert werden kann.

**37.** Vorrichtung nach einem der Ansprüche 29 bis 36,
**dadurch gekennzeichnet,**
**dass** zur Frequenzmessung bei der Bestimmung der Frequenz F eine Zeitmesseinrichtung und/oder ein Impulszählglied zur Zählung der von der Impulslichtquelle abgegebenen Lichtimpulse vorgesehen ist.

**38.** Vorrichtung nach Anspruch 37,
**dadurch gekennzeichnet,**
**dass** dem Impulszählglied ein Teilerelement vorgeschaltet ist, das die Anzahl der von der Impulslichtquelle abgegebenen Lichtimpulse in einem fest vorgegebenen Verhältnis teilt und das Teilerergebnis zur Auswertung weiterleitet.

**39.** Vorrichtung nach einem der Ansprüche 29 bis 38,
**dadurch gekennzeichnet,**
**dass** in der Vorrichtung ein Speicherelement vorgesehen ist, in dem die Messdauer bei der Frequenzmessung und/oder die bei der Frequenzmessung abzugebenden Lichtimpulse (09) als Festwert gespeichert werden können.

**40.** Vorrichtung nach einem der Ansprüche 29 bis 39,
**dadurch gekennzeichnet,**
**dass** die Vorrichtung einen konfokalen Optikfilter (21, 22, 23) umfasst, der in den Strahlengang der Reflektorlichtimpulse (11) einschaltbar ist, wobei der Optikfilter (21, 22, 23) Reflektorlichtimpulse (11), die im Nahbereich einer bestimmten Messebene (25) reflektiert werden, passieren lässt und wobei der Optikfilter (21, 22, 23) Reflektorlichtimpulse (11), die außerhalb des Nahbereichs der Messebene (25) reflektiert werden, abfiltert.

**41.** Vorrichtung nach Anspruch 40,
**dadurch gekennzeichnet,**
**dass** der konfokalen Optikfilter (21, 22, 23) ein Linsensystem (21, 22) zur Fokussierung der in der Messebene (25) liegenden Messpunkte und eine Lochblende (23) zur Ausfilterung aller nicht fokussierten Reflektorlichtimpulse (11) umfasst.

**42.** Vorrichtung nach Anspruch 40 oder 41,
**dadurch gekennzeichnet,**
**dass** das Linsensystem (21, 22) zur Fokussierung der auf der Messebene (25) liegenden Messpunkte ein Mikrolinsenelement (21) mit mehren in einer Ebene angeordneten Mikrolinsen umfasst.

**43.** Vorrichtung nach einem der Ansprüche 40 bis 42,

**dadurch gekennzeichnet,**
**dass** zur Einstellung der Messebene (25) des konfokalen Optikfilters (21, 22, 23) zumindest eine Linse (22) des Optikfilters verstellbar gelagert ist.

**44.** Vorrichtung nach einem der Ansprüche 29 bis 43, **dadurch gekennzeichnet,** **dass** die Vorrichtung (01) eine Fixierlichtquelle umfasst.

**Claims**

**1.** A method for determining the distance from a measurement point on a tissue surface (10, 13) of the eye (02), wherein

> a) using a pulsed light source (03), an initial light pulse (09a) is generated and directed onto the measurement point of the eye,
> b) a reflector light pulse (11) reflected on the tissue surface (10, 13) at the measurement point is intercepted by a light sensor (04),
> **characterised in that**
> c) a follow-up light pulse (09b) of the pulsed light source (03) is triggered by the detection of the reflector light pulse (11) at the light sensor (04),
> d) the pulsed light source (03) generates further follow-up light pulses (09b) depending on the detection of the reflector light pulses (11) caused by the respectively preceding follow-up light pulses (09b), and further oscillates at a frequency F1,
> e) the frequency F1 at which the pulsed light source (03) oscillates during reflection of the light pulses (09) at a first measurement point on a first tissue surface (10, 13) of the eye is measured directly or indirectly,
> and a frequency F2 at which the pulsed light source (03) oscillates during reflection of the light pulses (09) at a second measurement point on a second tissue surface (10, 13) of the eye is measured,
> f) the length difference XD by which the lengths X1 and X2 of the two ray paths differ during reflection of the light pulses (09) at the first measurement point and during reflection of the light pulses (09) at the second measurement point, with the lengths X1 and X2 of the ray paths starting from the pulsed light source (03) via the measurement points to the light sensor (04), is derived from the measured values of F1, F2 and the specific speed of light c at which the light propagates in the medium along the ray path, wherein the length difference XD represents the distance between the two tissue surfaces (10, 13), wherein the length difference XD between the two ray paths is derived according to the formula

$$XD = 0.5 \times c \times (1/F2 - 1/F1).$$

**2.** The method according to claim 1, **characterised in that** a second independent measurement method is used to determine the distance between a reference point and a measurement point on a tissue surface on the outer side of the eye, in particular on the anterior side of the cornea (13).

**3.** The method according to claim 2, **characterised in that** the distance determined using the second independent measurement method is used to pre-set and/or monitor the frequency measurement method.

**4.** The method according to claim 2, **characterised in that** the distance between the reference point and a reflecting measurement point on a tissue surface in the interior of the eye (02), in particular on the retina (10), is determined using the frequency measurement method, wherein the distance which exists between a tissue surface (10) in the interior of the eye (02) and a tissue surface (13) on the outer side of the eye (02) is derived by subtracting the distance determined using the second independent measurement method from the distance determined using the frequency measurement method.

**5.** The method according to any one of claims 1 to 4, **characterised in that** the reflector light pulse (11) is detected at the light sensor (04) by the light intensity measured by the light sensor (04) exceeding and/or falling below at least one predetermined trigger threshold (20).

**6.** The method according to claim 5, **characterised in that** the frequency measurement method is repeated in at least two measurement runs, wherein the light intensity of the light pulses (09) is varied between the different measurement runs.

**7.** The method according to claim 6, **characterised in that** the light intensity is varied by inserting a darkening element (17), in particular by displacing an optical wedge element, in the ray path of the light pulses (09).

**8.** The method according to any one of claims 1 to 7, **characterised in that** during the frequency measurement in order to determine the frequency F, the measurement duration is measured using a time measuring device and the light pulses (09) delivered during the measurement duration are counted using a pulse counting member.

9. The method according to any one of claims 1 to 7, **characterised in that** during the frequency measurement in order to determine the frequency F, the measurement duration is predefined as a fixed value and the light pulses (09) delivered during the fixed measurement duration are counted using the pulse counting member.

10. The method according to any one of claims 1 to 7, **characterised in that** during the frequency measurement in order to determine the frequency F, the frequency of the light pulses (09) to be delivered is predefined as a fixed value and the measurement duration required to deliver the light pulses (09) predefined as a fixed value is measured using the time measuring device.

11. The method according to any one of claims 1 to 10, **characterised in that** a confocal optical filter (21, 22, 23) is disposed in the ray path of the reflector light pulses (11), wherein the optical filter (21, 22, 23) transmits reflector light pulses (11) reflected in the near-field range of a specific measurement plane (25) of the optical filter and wherein said optical filter (21, 22, 23) filters out reflector light pulses (11) reflected outside the near-field range of said measurement plane (25).

12. The method according to claim 11, **characterised in that** the optical filter (21, 22, 23) is shifted continuously or in steps in such a manner that, starting from an initial position, the measurement plane (25) is shifted along the longitudinal axis of the ray path as far as an end position, wherein during the detection of reflector light pulses (11), a frequency measurement is carried out to determine the distance of the reflecting tissue surface.

13. The method according to claim 12, **characterised in that** the initial position or end position of the measurement plane lies before or on the anterior side of the cornea (13).

14. The method according to claim 12 or 13, **characterised in that** the initial position or end position of the measurement plane lies on or behind the retina (10).

15. The method according to any one of claims 1 to 14, **characterised in that**

the position of the eye is fixed by operation of a fixing light source.

16. The method according to any one of claims 1 to 15, **characterised in that** a flat height profile of the tissue surface is derived by measuring the distance of a plurality of adjacent measurement points on a tissue surface (10, 13) of the eye (02).

17. The method according to claim 16, **characterised in that** the distance of the plurality of adjacent measurement points on a tissue surface of the eye is measured by scanning the tissue surface, wherein a focused pulsed light beam is successively directed onto adjacent measurement points during the scanning.

18. The method according to claim 16, **characterised in that** in order to measure the distance of the plurality of adjacent measurement points on a tissue surface of the eye, a flat pulsed light beam is directed onto the tissue surface, wherein the pulsed light beams reflected at individual measurement points are detected separately from one another by a flat light sensor, in particular a flat CCD chip sensor.

19. The method according to any one of claims 16 to 18, **characterised in that** a flat height profile of the nerve fibre head is derived at the end of the optic nerve.

20. The method according to any one of claims 16 to 18, **characterised in that** a flat height profile of the cones on the retina is derived.

21. The method according to any one of claims 1 to 20, **characterised in that** at least one reflecting measurement point lies on a tissue surface on the outer side of the eye (02), in particular on the anterior side of the cornea (13).

22. The method according to any one of claims 1 to 20, **characterised in that** at least one reflecting measurement point lies on a tissue surface in the interior of the eye (02).

23. The method according to claim 22, **characterised in that** one measurement point lies on the retina (10).

24. The method according to claim 22, **characterised in that** one measurement point lies on the posterior side of the cornea (13) and/or on the anterior side of the lens body and/or on the posterior side of the lens body.

**25.** The method according to any one of claims 1 to 24, **characterised in that** the distance between one measurement point on the anterior side of the cornea (13) and one measurement point on the retina (10) is determined and is output as the axial length (14) of the eye (02).

**26.** The method according to any one of claims 1 to 25, **characterised in that** an averaged speed, averaged from the propagation velocity of light in the various media along the ray path between the pulsed light source (03) and the measurement point, is used as the specific speed of light c.

**27.** The method according to any one of claims 1 to 26, **characterised in that** the specific speed of light c is averaged from the propagation velocity of light in air and the averaged propagation velocity of light in the eye (02).

**28.** The method according to any one of claims 1 to 27, **characterised in that** the averaged propagation velocity of light in the eye (02) is averaged from the propagation velocity in the various media of the eye (02), in particular in the cornea (13) and/or in the anterior chamber and/or in the lens tissue and/or in the aqueous humor.

**29.** A device for determining the distance from a measurement point on a tissue surface (10, 13) of the eye (02), comprising

- at least one pulsed light source (03) for generating light pulses (09) which can be directed onto the measurement point,
- and at least one light sensor (04) for detecting reflector light pulses (11) reflected at the measurement point,
**characterised by**
- an electronic control system (06) which triggers further follow-up light pulses (09b) after detecting a reflector light pulse (11) and further oscillates at a frequency F1,
- a frequency measuring device for direct or indirect measurement of the frequency F1 which oscillates at a first measurement point on a first tissue surface (10, 13) of the eye and of a frequency F2 which oscillates at a second measurement point on a second tissue surface (10, 13) of the eye,
- a computation unit whereby the length difference $X_D$ by which the lengths X1 and X2 of the two ray paths differ during reflection of the light pulses (09) at the first measurement point and during reflection of the light pulses (09) at the second measurement point, with the lengths X1 and X2 of the ray paths starting from the pulsed light source (03) via the measurement points to the light sensor (04), is derived from the measured values of F1, F2 and the specific speed of light c at which the light propagates in the medium along the ray path, wherein the length difference XD represents the distance between the two tissue surfaces (10, 13), wherein the length difference XD between the two ray paths is derived according to the formula

$$XD = 0.5 \times c \times (1/F2 - 1/F1).$$

**30.** The device according to claim 29, **characterised in that** the pulsed light source (03) and the light sensor (04) are arranged in the device (01) in such a manner that the length X of the ray path between the pulsed light source (03) and the measurement point exactly corresponds to the length Y of the ray path between the measurement point and the light sensor (04).

**31.** The device according to claim 29 or 30, **characterised in that** the pulsed light source (03) is embodied in the manner of a laser light source, in particular in the manner of a laser diode.

**32.** The device according to any one of claims 29 to 31, **characterised in that** the light sensor (04) is embodied in the manner of a photodiode or in the manner of an in particular flat CCD chip sensor.

**33.** The device according to any one of claims 29 to 32, **characterised in that** a second independent measuring device (12) is integrated in the device (01), whereby the distance between a reference point and a measurement point on a tissue surface on the outer side of the eye, in particular on the anterior side of the cornea, can be determined.

**34.** The device according to claim 33, **characterised in that** the second independent measuring device (12) is embodied in the manner of a triangulation measuring device.

**35.** The device according to any one of claims 29 to 34, **characterised in that** the light intensity of the light sensor (04) is evaluated in a light intensity trigger element, wherein the reflector light pulse (11) is detected at the light sensor (04) by the fact that the light intensity measured by the light sensor exceeds or falls below a predefined trigger threshold (20).

36. The device according to any one of claims 29 to 35, **characterised in that** the device (01) comprises a darkening element (17), in particular an optical wedge element, whereby the light intensity of the light pulses (09) can be varied.

37. The device according to any one of claims 29 to 36, **characterised in that** for measuring the frequency when determining the frequency F, a time measuring device and/or a pulse counting member for counting the pulses delivered by the pulsed light source is provided.

38. The device according to claim 37, **characterised in that** the pulse counting member is preceded by a divider element which divides the number of light pulses delivered by the pulsed light source in a fixedly predefined ratio and passes on the divider result for evaluation.

39. The device according to any one of claims 29 to 38, **characterised in that** a storage element is provided in the device, in which the measurement duration during the frequency measurement and/or the light pulses (09) to be delivered during the frequency measurement can be stored as a fixed value.

40. The device according to any one of claims 29 to 39, **characterised in that** the device comprises a confocal optical filter (21, 22, 23) which can be inserted into the ray path of the reflector light pulses (11), wherein the optical filter (21, 22, 23) transmits reflector light pulses (11) reflected in the near-field range of a specific measurement plane (25) and wherein said optical filter (21, 22, 23) filters out reflector light pulses (11) reflected outside the near-field range of said measurement plane (25).

41. The device according to claim 40, **characterised in that** the confocal optical filter (21, 22, 23) comprises a lens system (21, 22) for focusing the measurement points located in the measurement plane (25) and an aperture (23) for filtering out all non-focused reflector light pulses (11).

42. The device according to claim 40 or 41, **characterised in that** the lens system (21, 22) for focusing the measurement points located on the measurement plane (25) comprises a microlens element (21) provided with a plurality of microlenses disposed in one plane.

43. The device according to any one of claims 40 to 42, **characterised in that** at least one lens (22) of the optical filter is adjustably mounted to adjust the measurement plane (25) of the confocal optical filter (21, 22, 23).

44. The device according to any one of claims 29 to 43, **characterised in that** the device (01) comprises a fixing light source.

**Revendications**

1. Procédé de détermination de la distance par rapport à un point de mesure sur une surface de tissu (10, 13) de l'oeil (02), dans lequel

a) une première impulsion lumineuse (09a) est générée au moyen d'une source lumineuse pulsée (03) et dirigée sur le point de mesure de l'oeil,
b) une impulsion lumineuse reflétée (11) qui se reflète sur la surface de tissu (10, 13) au point de mesure est interceptée par un capteur de lumière (04), **caractérisé en ce que**
c) une impulsion lumineuse consécutive (09b) de la source lumineuse pulsée (03) est déclenchée par la détection de l'impulsion lumineuse reflétée (11) au capteur de lumière (04),
d) en fonction de la détection des impulsions lumineuses reflétées (11) qui sont provoquées par les impulsions lumineuses consécutives (09b) respectivement précédentes, la source lumineuse pulsée (03) génère d'autres impulsions lumineuses consécutives (09b) et en outre elle oscille à une fréquence F1,
e) la fréquence F1 à laquelle la source lumineuse pulsée (03) oscille pendant la réflexion des impulsions lumineuses (09) à un premier point de mesure sur une première surface de tissu (10, 13) de l'oeil est mesurée directement ou indirectement,
et une fréquence F2 à laquelle la source lumineuse pulsée (03) oscille pendant la réflexion des impulsions lumineuses (09) à un deuxième point de mesure sur une deuxième surface de tissu (10, 13) de l'oeil est mesurée,
f) la différence de longueur XD par laquelle les longueurs X1 et X2 des deux trajets du faisceau se distinguent l'une de l'autre pendant la réflexion des impulsions lumineuses (09) au premier point de mesure et pendant la réflexion des impulsions lumineuses (09) au deuxième point de mesure, avec les longueurs X1 et X2 des trajets du faisceau, en partant de la source lumineuse pulsée (03), en passant par les points de mesure jusqu'au capteur de lumière (04), est dérivée des valeurs mesurées de F1, F2 et la vitesse de la lumière spécifique c à laquelle la lumière se propage dans le milieu le long du trajet du faisceau, dans lequel la différence de longueur XD représente la distance entre les deux surfaces de tissu (10, 13), dans lequel la différence de longueur XD entre les deux trajets du faisceau est dérivée selon la formule

$$XD = 0.5 \times c \times (1/F2 - 1/F1).$$

2. Procédé selon la revendication 1,
**caractérisé en ce que**
un deuxième procédé de mesure indépendant est utilisé pour déterminer la distance entre un point de référence et un point de mesure sur une surface de tissu sur le côté extérieur de l'oeil, en particulier sur la face antérieure de la cornée (13).

3. Procédé selon la revendication 2,
**caractérisé en ce que**
la distance qui est déterminée au moyen du deuxième procédé de mesure indépendant est utilisée pour prérégler et/ou pour contrôler le procédé de mesure de la fréquence.

4. Procédé selon la revendication 2,
**caractérisé en ce que**
la distance entre le point de référence et un point de mesure reflétant sur une surface de tissu à l'intérieur de l'oeil (02), en particulier sur la rétine (10), est déterminée au moyen du procédé de mesure de la fréquence, dans lequel la distance qui existe entre une surface de tissu (10) à l'intérieur de l'oeil (02) et une surface de tissu (13) sur le côté extérieur de l'oeil (02) est dérivée en soustrayant la distance déterminée au moyen du deuxième procédé indépendant de la distance déterminée au moyen du procédé de mesure de la fréquence.

5. Procédé selon une des revendications 1 à 4,
**caractérisé en ce que**
l'impulsion lumineuse reflétée (11) est détectée au capteur de lumière (04) du fait que l'intensité lumineuse mesurée par le capteur de lumière (04) passe au-dessus et/ou en-dessous d'au moins un seuil de déclenchement (20) prédéfini.

6. Procédé selon la revendication 5,
**caractérisé en ce que**
le procédé de mesure de la fréquence est répété dans au moins deux tours de mesure, dans lequel l'intensité lumineuse des impulsions lumineuses (09) est modifiée entre les différents tours de mesure.

7. Procédé selon la revendication 6,

**caractérisé en ce que**
l'intensité lumineuse est modifiée en insérant un élément d'obscurcissement (17), en particulier en réglant un élément cale optique, dans le trajet du faisceau des impulsions lumineuses (09).

8. Procédé selon une des revendications 1 à 7,
**caractérisé en ce que**
pendant la mesure de la fréquence pour déterminer la fréquence F, la durée de la mesure est mesurée au moyen d'un dispositif de chronométrage et les impulsions lumineuses (09) émises pendant la durée de la mesure sont comptées au moyen d'un élément pour compter des impulsions.

9. Procédé selon une des revendications 1 à 7,
**caractérisé en ce que**
pendant la mesure de la fréquence pour déterminer la fréquence F, la durée de la mesure est prédéfinie comme un valeur fixe et les impulsions lumineuses (09) émises pendant la durée de la mesure prédéfinie sont comptées au moyen de l'élément pour compter des impulsions.

10. Procédé selon une des revendications 1 à 7,
**caractérisé en ce que**
pendant la mesure de la fréquence pour déterminer la fréquence F, la fréquence des impulsions lumineuses (09) à émettre est prédéfinie comme un valeur fixe et la durée de la mesure qui est nécessaire pour émettre les impulsions lumineuses (09) prédéfinies comme un valeur fixe est mesurée au moyen du dispositif de chronométrage.

11. Procédé selon une des revendications 1 à 10,
**caractérisé en ce que**
un filtre optique confocal (21, 22, 23) est disposé dans le trajet du faisceau des impulsions lumineuses reflétées (11), dans lequel le filtre optique (21, 22, 23) transmet des impulsions lumineuses reflétées (11) qui se reflètent dans la zone de champ proche d'un plan de mesure (25) spécifique du filtre optique et dans lequel le filtre optique (21, 22, 23) filtre des impulsions lumineuses reflétées (11) qui se reflètent à l'écart de la zone de champ proche du plan de mesure (25).

12. Procédé selon la revendication 11,
**caractérisé en ce que**
le filtre optique (21, 22, 23) est réglé continuellement ou progressivement de telle manière que, en partant d'une position initiale, le plan de mesure (25) est réglé le long de l'axe longitudinal du trajet du faisceau jusqu'à une position finale, dans lequel, après la détection d'impulsions lumineuses reflétées (11), une mesure de la fréquence est effectuée pour déterminer la distance du surface de tissu reflétante.

**13.** Procédé selon la revendication 12,
**caractérisé en ce que**
la position initiale ou la position finale du plan de mesure se situe devant ou sur la face antérieure de la cornée (13).

**14.** Procédé selon la revendication 12 ou 13,
**caractérisé en ce que**
la position initiale ou la position finale du plan de mesure se situe sur ou derrière la rétine (10).

**15.** Procédé selon une des revendications 1 à 14,
**caractérisé en ce que**
la position de l'oeil est fixée en actionnant une source lumineuse fixante.

**16.** Procédé selon une des revendications 1 à 15,
**caractérisé en ce que**
un profil de hauteur plat de la surface de tissu est dérivé en mesurant la distance de plusieurs points de mesure adjacents sur une surface de tissu (10, 13) de l'oeil (02).

**17.** Procédé selon la revendication 16,
**caractérisé en ce que**
la distance des plusieurs points de mesure adjacents sur une surface de tissu de l'oeil est mesurée en balayant la surface de tissu, dans lequel un faisceau lumineux pulsé et focalisé est dirigé sur des points de mesure adjacents l'un après l'autre pendant le balayage.

**18.** Procédé selon la revendication 16,
**caractérisé en ce que**
pour mesurer la distance des plusieurs points de mesure adjacents sur une surface de tissu de l'oeil, un faisceau lumineux pulsé et plat est dirigé sur la surface de tissu, dans lequel les faisceaux lumineux pulsés qui se reflètent à des points de mesure individuels sont détectés séparément par un capteur de lumière plat, en particulier par un capteur CCD plat.

**19.** Procédé selon une des revendications 16 à 18,
**caractérisé en ce que**
un profil de hauteur plat de la tête des fibres nerveuses est dérivé à l'extrémité du nerf optique.

**20.** Procédé selon une des revendications 16 à 18,
**caractérisé en ce que**
un profil de hauteur plat des cônes de la rétine est dérivé.

**21.** Procédé selon une des revendications 1 à 20,
**caractérisé en ce que**
au moins un point de mesure reflétant se situe sur une surface de tissu sur le côté extérieur de l'oeil (02), en particulier sur la face antérieure de la cornée (13).

**22.** Procédé selon une des revendications 1 à 20,
**caractérisé en ce que**
au moins un point de mesure reflétant se situe sur une surface de tissu à l'intérieur de l'oeil (02).

**23.** Procédé selon la revendication 22,
**caractérisé en ce que**
un point de mesure se situe sur la rétine (10).

**24.** Procédé selon la revendication 22,
**caractérisé en ce que**
un point de mesure se situe à l'arrière de la cornée (13) et/ou sur la face antérieure du corps lenticulaire et/ou à l'arrière du corps lenticulaire.

**25.** Procédé selon une des revendications 1 à 24,
**caractérisé en ce que**
la distance entre un point de mesure sur la face antérieure de la cornée (13) et un point de mesure sur la rétine (10) est déterminée et émise comme la longueur axiale (14) de l'oeil (02).

**26.** Procédé selon une des revendications 1 à 25,
**caractérisé en ce que**
une vitesse moyenne, la moyenne calculée à partir de la vitesse de propagation de la lumière dans les milieux différents le long du trajet du faisceau entre la source lumineuse pulsée (03) et le point de mesure, est utilisée comme la vitesse de la lumière spécifique c.

**27.** Procédé selon une des revendications 1 à 26,
**caractérisé en ce que**
la moyenne de la vitesse de la lumière spécifique c est calculée à partir de la vitesse de propagation de la lumière dans l'air et de la moyenne de la vitesse de propagation de la lumière dans l'oeil (02).

**28.** Procédé selon une des revendications 1 à 27,
**caractérisé en ce que**
la moyenne de la vitesse de propagation de la lumière dans l'oeil (02) est calculée à partir de la vitesse de propagation dans les milieux différents de l'oeil (02), en particulier dans la cornée (13) et/ou dans la chambre antérieure et/ou dans le tissu de la lentille et/ou dans l'humeur aqueuse.

**29.** Dispositif de détermination de la distance par rapport à un point de mesure sur une surface de tissu (10, 13) de l'oeil (02), comprenant

- au moins une source lumineuse pulsée (03) pour générer des impulsions lumineuses (09) qui peuvent être dirigées sur le point de mesure,
- et au moins un capteur de lumière (04) pour détecter des impulsions lumineuses reflétées (11) qui se reflètent au point de mesure,
**caractérisé par**

- une électronique de commande (06) qui déclenche d'autres impulsions lumineuses consécutives (09b) après la détection d'une impulsion lumineuse reflétée (11) et qui oscille, en outre, à une fréquence F1,

- un dispositif de mesure de la fréquence pour mesurer directement ou indirectement la fréquence F1 qui oscille à un premier point de mesure sur une première surface de tissu (10, 13) de l'oeil et une fréquence F2 qui oscille à un deuxième point de mesure sur une deuxième surface de tissu (10, 13) de l'oeil,

- une unité de calcul au moyen de laquelle la différence de longueur XD par laquelle les longueurs X1 et X2 des deux trajets du faisceau se distinguent l'une de l'aitre pendant la réflexion des impulsions lumineuses (09) au premier point de mesure et pendant la réflexion des impulsions lumineuses (09) au deuxième point de mesure, avec les longueurs X1 et X2 des trajets du faisceau, en partant de la source lumineuse pulsée (03), en passant par les points de mesure jusqu'au capteur de lumière (04), est dérivée des valeurs mesurées de F1, F2 et la vitesse de la lumière spécifique c à laquelle la lumière se propage dans le milieu le long du trajet du faisceau, dans lequel la différence de longueur XD représente la différence entre les deux surfaces de tissu (10, 13), dans lequel la différence de longueur XD entre les deux trajets du faisceau est dérivée selon la formule

$$XD = 0.5 \times c \times (1/F2 - 1/F1).$$

30. Dispositif selon la revendication 29,
**caractérisé en ce que**
la source lumineuse pulsée (03) et le capteur de lumière (04) sont disposés dans le dispositif (01) de telle manière que la longueur X du trajet du faisceau entre la source lumineuse pulsée (03) et le point de mesure correspond à la longueur Y du trajet du faisceau entre le point de mesure et le capteur de lumière (04) exactement.

31. Dispositif selon la revendication 29 ou 30,
**caractérisé en ce que**
la source lumineuse pulsée (03) est conçue sous forme d'une source lumineuse laser, en particulier sous forme d'une diode laser.

32. Dispositif selon une des revendications 29 à 31,
**caractérisé en ce que**
le capteur de lumière (04) est conçue sous forme d'une photodiode ou sous forme d'un capteur CCD, en particulier sous forme d'un capteur CCD plat.

33. Dispositif selon une des revendications 29 à 32,
**caractérisé en ce que**
un deuxième dispositif de mesure (12) indépendant est intégré dans le dispositif (01), au moyen duquel la distance entre un point de référence et un point de mesure sur une surface de tissu sur le côté extérieur de l'oeil, en particulier sur la face antérieure de la cornée (13), peut être déterminée.

34. Dispositif selon la revendication 33,
**caractérisé en ce que**
le deuxième dispositif de mesure (12) indépendant est conçu sous forme d'un dispositif de mesure à triangulation.

35. Dispositif selon une des revendications 29 à 34,
**caractérisé en ce que**
l'intensité lumineuse du capteur de lumière (04) est évaluée dans un élément de déclenchement d'intensité lumineuse, dans lequel l'impulsion lumineuse reflétée est détectée au capteur de lumière (04) du fait que l'intensité lumineuse mesurée par le capteur de lumière (04) passe au-dessus et/ou en-dessous d'au moins un seuil de déclenchement (20) prédéfini.

36. Dispositif selon une des revendications 29 à 35,
**caractérisé en ce que**
le dispositif (01) comprend un élément d'obscurcissement (17), en particulier un élément cale optique, au moyen duquel l'intensité lumineuse des impulsions lumineuses (09)peut être modifiée.

37. Dispositif selon une des revendications 29 à 36,
**caractérisé en ce que**
pour la mesure de la fréquence pour déterminer la fréquence F, un dispositif de chronométrage et/ou un élément pour compter des impulsions pour le compte des impulsions lumineuses (09) émises par la source lumineuse pulsée est prévu.

38. Dispositif selon la revendication 37,
**caractérisé en ce que**
un élément de division précède l'élément pour compter des impulsions et divise le nombre d'impulsions lumineuses émises par la source lumineuse pulsée selon un rapport prédéfini de manière fixe avant de transmettre le résultat partiel afin qu'il soit evalué.

39. Dispositif selon une des revendications 29 à 38,
**caractérisé en ce que**
un élément de mémoire est prévu dans le dispositif sur lequel la durée de la mesure pendant la mesure de la fréquence et/ou les impulsions lumineuses (09) à émettre pendant la mesure de la fréquence peuvent être stockées comme un valeur fixe.

40. Dispositif selon une des revendications 29 à 39,
**caractérisé en ce que**

le dispositif comprend un filtre optique confocal (21, 22, 23) qui peut être inséré dans le trajet du faisceau des impulsions lumineuses reflétées (11), dans lequel le filtre optique (21, 22, 23) transmet des impulsions lumineuses reflétées (11) qui se reflètent dans la zone de champ proche d'un plan de mesure (25) spécifique du filtre optique et dans lequel le filtre optique (21, 22, 23) filtre des impulsions lumineuses reflétées (11) qui se reflètent à l'écart de la zone de champ proche du plan de mesure (25).

**41.** Dispositif selon la revendication 40,
   **caractérisé en ce que**
   le filtre optique confocal (21, 22, 23) comprend un système de lentilles (21, 22) pour focaliser les points de mesure situés dans le plan de mesure (25) et un diaphragme (23) pour filtrer toutes les impulsions lumineuses reflétées (11) non focalisées.

**42.** Dispositif selon la revendication 40 ou 41,
   caracerisé en ce que
   le système de lentilles (21, 22) pour focaliser les points de mesure situés dans le plan de mesure (25) comprend un élément de microlentilles (21) avec plusieurs microlentilles disposées dans un plan.

**43.** Dispositif selon une des revendications 40 à 42,
   **caractérisé en ce que**
   au moins une lentille (22) du filtre optique est montée de manière réglable pour régler le plan de mesure (25) du filtre optique confocal (21, 22, 23).

**44.** Dispositif selon une des revendications 29 à 43,
   **caractérisé en ce que**
   le dispositif (01) comprend une source lumineuse fixante.

Fig. 1

Fig. 2

Fig. 3

Fig. 4

Fig. 5

Fig. 6

Fig. 7

Fig. 8

Fig. 9

Fig. 10

Fig. 11